(19) Europäisches Patentamt
European Patent Office
Office européen des brevets

(11) Numéro de publication: **0 317 416 A1**

(12) **DEMANDE DE BREVET EUROPEEN**

(21) Numéro de dépôt: **88402852.3**

(22) Date de dépôt: **14.11.88**

(51) Int. Cl.[4]: **C 07 D 221/08**
C 07 D 471/04,
C 07 D 471/14,
C 07 D 495/04, A 61 K 31/47,
A 61 K 31/475, C 07 D 221/18

(30) Priorité: **16.11.87 FR 8715791**

(43) Date de publication de la demande:
**24.05.89 Bulletin 89/21**

(84) Etats contractants désignés:
**AT BE CH DE ES FR GB GR IT LI LU NL SE**

(71) Demandeur: **CENTRE NATIONAL DE LA RECHERCHE SCIENTIFIQUE**
**15, quai Anatole France**
**F-75007 Paris (FR)**

(72) Inventeur: **Bisagni, Emile Raymond Barthélémy**
**16 Rue Bossuet**
**F-91400 Orsay (FR)**

**Grelet épouse Rautureau, Marilys**
**Résidence les Chandeliers 99 Aven. Général Leclerc**
**F-91120 Palaiseau (FR)**

(74) Mandataire: **Phélip, Bruno et al**
**c/o Cabinet Harlé & Phélip 21, rue de La Rochefoucauld**
**F-75009 Paris (FR)**

(54) **Procédé de préparation de chloro-1 alkyl-5 isoquinoleines condensées avec des groupes aromatiques, les nouveaux produits obtenus, et leur application comme médicaments.**

(57) Pour préparer le composé (I), on effectue une lithiation du composé (II), on fait réagir, sur le composé (III) formé, un aldéhyde (IV), ce qui conduit à l'alcool secondaire (V) que l'on soumet à une réduction conduisant au composé (VI) qui est alors soumis à une réaction d'hydrolyse (de la fonction dioxolanne)-cyclisation-déshydratation. On peut faire suivre par une réaction de substitution nucléophile du chlore. Les composés (a) ci-après présentent une activité antitumorale.

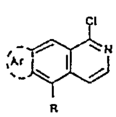
(I)

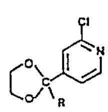
(II)

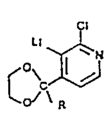
(III)

ArCHO
(IV)

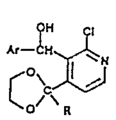
(V)

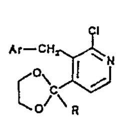
(VI)

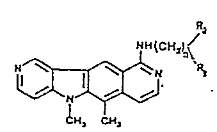
(a)

R = alkyle en $C_1$-$C_3$ ; Ar = groupe aromatique ou polyaromatique ; $R_1$ et $R_2$ = H ou alkyle en $C_1$-$C_3$ ; et n = 2 à 4.

EP 0 317 416 A1

## Description

### PROCEDE DE PREPARATION DE CHLORO-1 ALKYL-5 ISOQUINOLEINES CONDENSEES AVEC DES GROUPES AROMATIQUES, LES NOUVEAUX PRODUITS OBTENUS, ET LEUR APPLICATION COMME MEDICAMENTS.

La présente invention concerne un procédé de préparation de chloro-1 alkyl-5 isoquinoléines condensées suivant leur liaison [g] avec des groupes aromatiques, ces isoquinoléines étant représentées par la formule (I) ci-après :

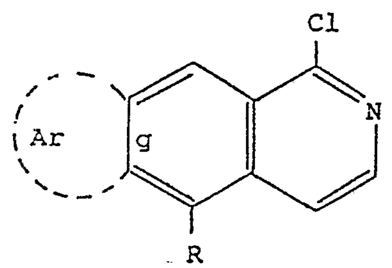

(I)

où :

- R représente un groupe alkyle en $C_1$ à $C_3$ ; et
- Ar représente un groupe aromatique ou hétéroaromatique, mono- ou polycyclique, éventuellement substitué,

ainsi que les composés correspondants obtenus par substitution nucléophile du chlore du noyau pyridinique.

Certains de ces composés sont déjà connus. On peut citer notamment le composé (Ia) :

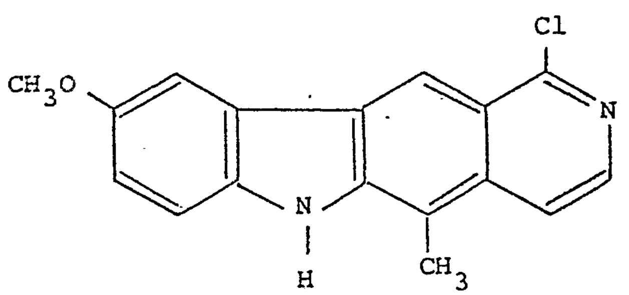

(Ia)

qui est un intermédiaire de synthèse de substances anticancéreuses décrites dans la demande de brevet français n° 2 436 786 et qui est obtenu par un procédé complexe, puisqu'il comporte douze étapes, à partir de :

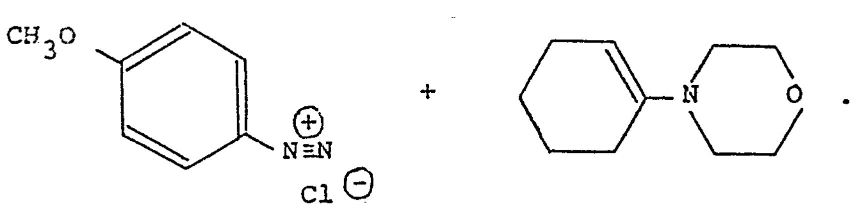

On peut citer également le composé (Ib) :

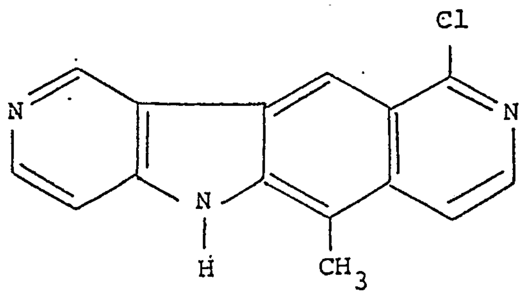

(Ib)

qui est un intermédiaire de synthèse de substances anticancéreuses décrites dans la demande de brevet français n° 2 387 229, et qui est obtenu, avec un faible rendement, par un procédé en onze étapes à partir du composé :

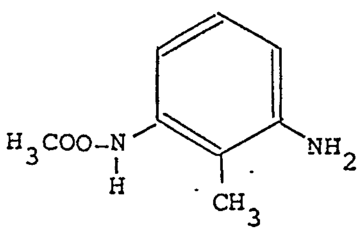

Le procédé selon la présente invention n'implique qu'un nombre d'étapes limité, et il permet d'obtenir, avec de bons rendements, des chloro-1 alkyl-5 isoquinoléines condensées avec des groupes aromatiques et hétéroaromatiques.

Ce procédé est essentiellement, caractérisé par le fait qu'il comprend les étapes consistant à :

(a) effectuer une lithiation du composé de formule (II) :

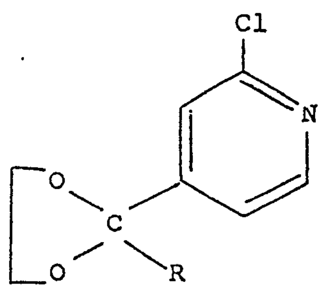

(II)

où R est tel que défini ci-dessus,

de façon à former le composé de formule (III) :

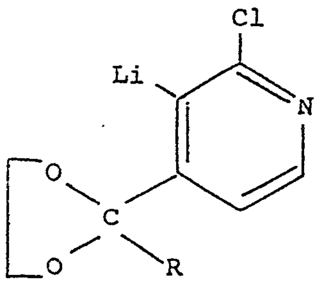

(III)

(b) faire réagir un aldéhyde de formule (IV) :

ArCHO (IV)

où Ar est tel que défini ci-dessus,

avec le composé de formule (III) précité, ce qui conduit à l'alcool secondaire de formule (V) :

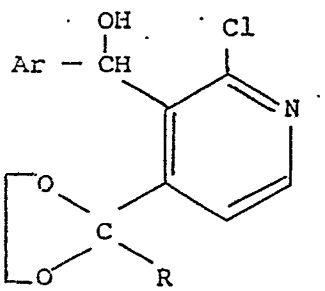

(V)

(c) on soumet l'alcool secondaire de formule (V) à une réduction de la fonction alcool secondaire en groupe méthylénique, ce qui conduit au composé de formule (VI) :

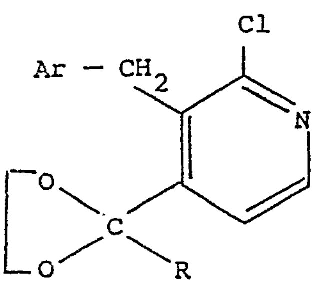

(VI) ; et

et

(d) on soumet le composé de formule (VI) à une réaction d'hydrolyse (de la fonction dioxolanne)-cyclisation-déshydratation, ce qui conduit au composé de formule (I) : et l'on peut faire suivre par une réaction de substitution nucléophile du chlore.

Le composé de formule (II) est préparé selon le mode opératoire décrit par J.L. Lamattina dans ≪J. Het. Chem. <u>30</u> p. 353 (1983)≫ pour l'acétyl-4 chloro-2 pyridine éthylène glycol acétal (composé de formule (II) où R représente $CH_3$).

L'étape (a) de lithiation du composé de formule (II), conduisant au composé de formule (III), est effectuée comme pour celle d'autres dérivés de la pyridine, par action du diisopropylamidure de lithium dans le tétrahydrofuranne anhydre, à basse température.

L'étape (b) du procédé de la présente invention, à savoir la condensation avec l'aldéhyde de formule (IV) a lieu, de façon classique, par addition de ce dernier à la solution, par exemple dans le tétrahydrofuranne anhydre, du composé organolithien de formule (III).

On peut condenser, sur le composé organolithien de formule (III), de nombreux aldéhydes aromatiques et hétéroaromatiques, substitués ou non substitués, par exemple avec les dérivés du pyrrole ou de l'indole

protégés par des groupes tels que les groupes alkyle, arylalkyle, alkylcarbonyle, alkyle et arylsulfonyle ou carbamoyle sur leur sommet 1. Ainsi, on peut citer l'application de ce procédé à la préparation de composés de formule (I) dans laquelle Ar représente :

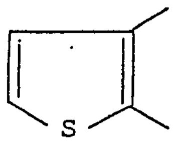 ; 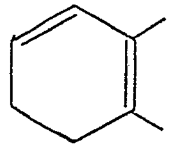 ; 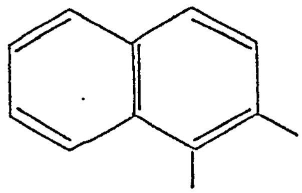 ; 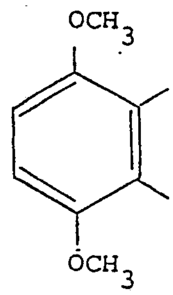 ;

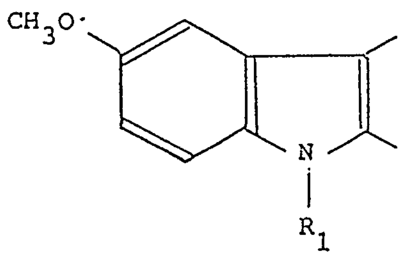 ; 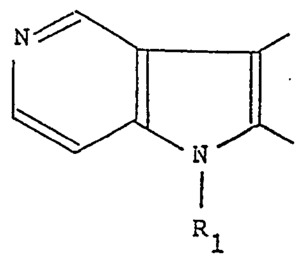 .

$R_1$ étant tel que défini ci-après.

La réduction de la fonction alcool secondaire en groupe méthylénique, qui est la réaction de l'étape (c) du procédé selon la présente invention, peut être effectuée par tout moyen connu pour réduire les benzhydrols, choisi de telle sorte qu'il n'y ait pas réduction de la fonction acétal ni élimination du chlore du noyau pyridine. Le triéthylsilane en solution dans l'acide trifluoracétique convient pour cette réduction.

La réaction de l'étape (d) est une triple réaction qui peut être schématisée comme suit :

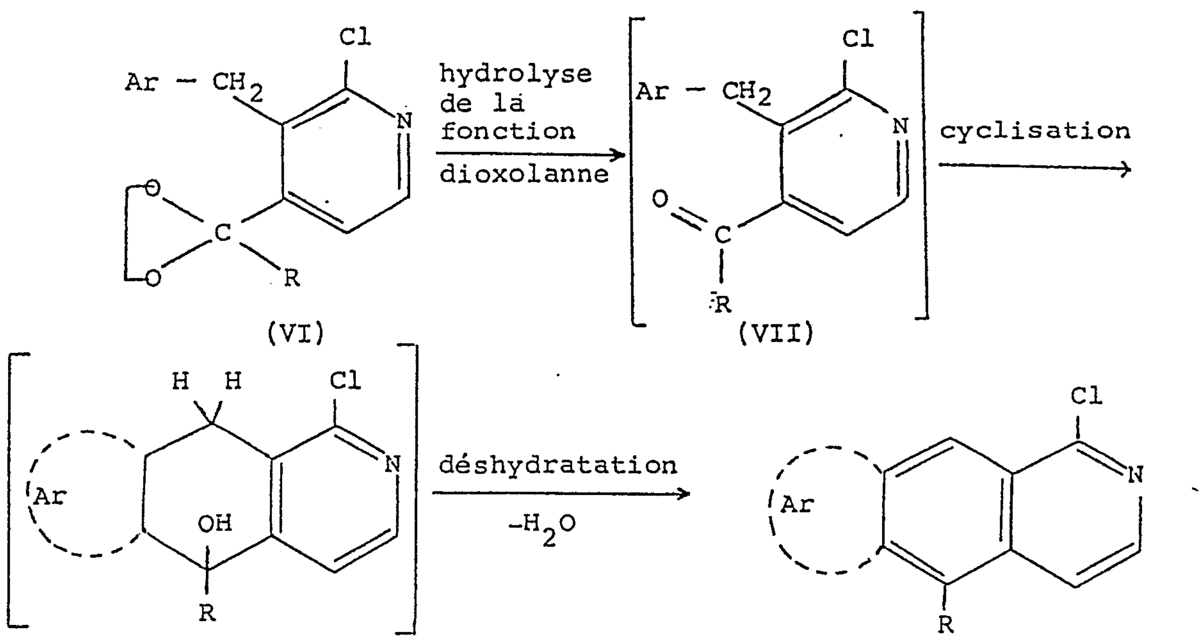


(VI) (VII) (VIII) (I)

Les composés de formule (VII) et (VIII) ne sont pas isolés. Par ailleurs, il n'est pas obligatoire d'isoler le

composé de formule (VI).

La fonction dioxolanne est aisément hydrolysable en milieu acide et il apparaît simultanément le produit résultant de la cyclodéshydratation de la cétone correspondante. Autrement dit, le composé de formule (VI) peut être cyclisé en milieu aqueux par action d'un acide fort, tel que $H_2SO_4$ ou HCl, de préférence, à une température comprise entre 40°C et 70°C.

En particulier, le procédé selon la présente invention s'applique avantageusement à la préparation des chloro-1 alkyl-5 isoquinoléines condensées suivant leur liaison [g] avec des hétérocycles azotés, susbtitués sur l'azote, et, notamment, des dérivés de l'indole et homologues, les composés obtenus répondant à la formule (I') :

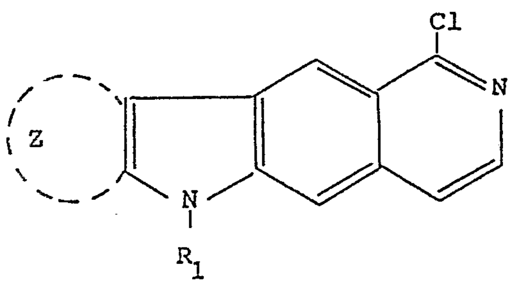

(I')

où :

- R représente un groupe alkyle en $C_1$ à $C_3$ ;
- $R_1$ représente un groupe alkyle en $C_1$ à $C_3$ ou un groupement protecteur éliminable ; et
- Z représente un groupe aromatique ou hétéroaromatique, substitué ou non substitué, tel que :

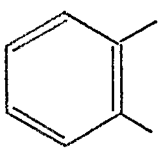 , 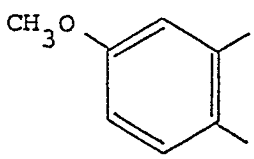 , 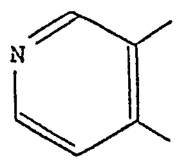

Dans ce cas, on fait réagir, comme composé de formule (IV), l'aldéhyde de formule (IVa) N-protégé :

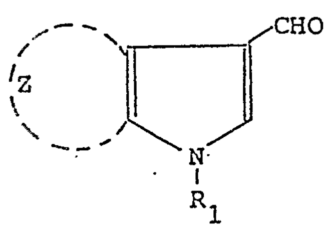

(IVa)

où $R_1$ est tel que défini ci-dessus.

La présente invention concerne également les produits obtenus par la mise en ouvre de ce procédé, et à titre de produits nouveaux, les produits de formule (I) dans laquelle Ar représente :

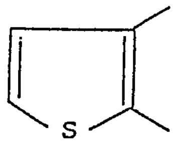
,
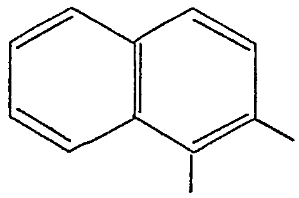
,
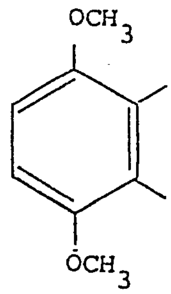

et notamment
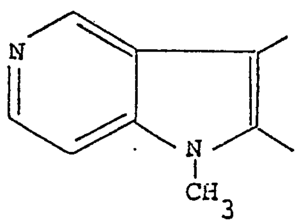
.

En particulier, les produits de formule

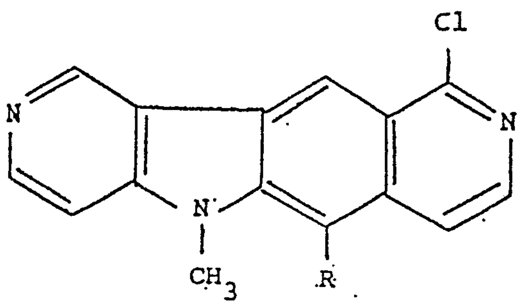

peuvent conduire par substitution nucléophile du chlore du noyau pyridinique à de nombreux composés, par exemple, à des amines. On peut ainsi mentionner les composés de formule :

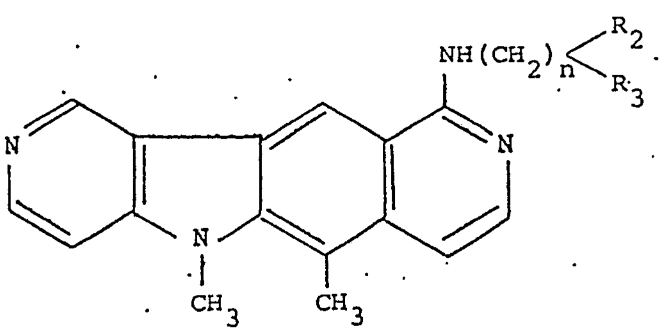

où :

- n = 2 à 4 ; et
- $R_2$ et $R_3$ représentent chacun, indépendamment l'un de l'autre, H ou alkyle en $C_1$-$C_3$.

Ces composés, qui sont préparés par action des diamines voulues sur le dérivé chloré correspondant obtenu par le procédé de l'invention, et obtenus sous la forme des bases libres ou des sels pharmacologiquement acceptables correspondants (par exemple maléates ou chlorhydrates) ont une activité anticancéreuse au moins comparable à celle de leurs homologues non alkylés sur l'azote indolique.

La présente invention porte également sur ces agents thérapeutiques pour le traitement de divers cancers dont les leucémies, ainsi que sur des compositions médicamenteuses les contenant en combinaison avec un

support pharmaceutiquement acceptable, adapté à une administration des compositions par la voie intraveineuse ou la voie intrapéritonéale, comme le soluté physiologique.

Le médecin déterminera le dosage de ces agents thérapeutiques qui conviendra le mieux. Ce dosage variera selon la forme d'administration et le composé particulier choisi et, par ailleurs, il variera avec le patient particulier soumis au traitement. Les composés sont utiles de la même manière que d'autres agents analogues, et le niveau de dosage sera du même ordre que pour ces derniers.

On décrira dans ce qui suit des exemples de mise en oeuvre du procédé de l'invention pour l'obtention de chloro-1 méthyl-5 isoquinoléines condensées par leur liaison [g] à des noyaux aromatiques, nouvelles ou précédemment décrites, ainsi que de préparation de certains de leurs dérivés ayant une activité pharmacologique.

Les points de fusion ont été pris au banc Kofler. Les spectres de RMN $^1H$ ont été enregistrés sur un appareil ≪Varian XL 100≫, dans les solvants indiqués, et les déplacements chimiques sont donnés en ppm par rapport au $(CH_3)_4Si$.

EXEMPLE 1

a) Première étape :

Préparation du dioxolanne de l'acétyl-4 chloro-2 pyridine :

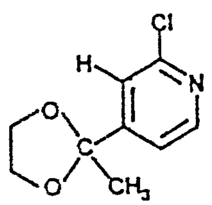

Ce composé a été prépàré comme indiqué par Lamattina J.L. dans ≪J. Het. Chem. 1983, 20, 353≫ qui le décrit comme un liquide. Distillé à 128° C sous 1333,22 Pa (10 mmHg), il cristallise en donnant un solide qui fond aux environs de 30° C.

b) Deuxième étape

Préparation des trois acétyl-4 chloro-2 (α-aryl α-hydroxy) méthyl-3 pyridine éthylène glycol acétals :

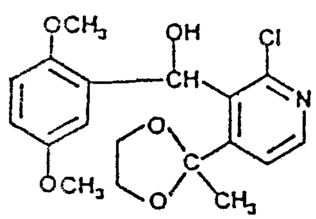

(A)

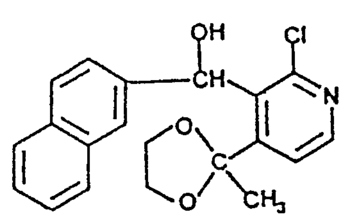

(B)

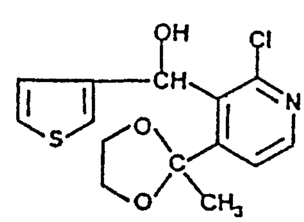

(C)

Dans un tricol de 250 ml, séché à 120° C et maintenu sous argon, on introduit le tétrahydrofuranne (THF, 50 ml) fraîchement distillé sur le mélange benzophénone + sodium. Après avoir refroidi à 0° C, le butyllithium (6,9 ml de la solution commerciale 1,6 M, 11 mmoles) et la diisopropylamine distillée sur hydrure de calcium (1,54 ml, 11 mmoles), sont ajoutés successivement. L'ensemble est agité à 0° C, pendant 1 heure, puis refroidi à -70° C. En continuant à refroidir à cette dernière température, le dioxolanne obtenu à la première étape (1,995 g, 10 mmoles), en solution dans le THF (10 ml), est alors ajouté, goutte à goutte. Au bout de 30 minutes après la fin de l'addition, on observe l'apparition d'un précipité jaune, et le mélange hétérogène se transforme progressivement en un gel. Après 4 heures à -70° C, l'aldéhyde choisi (10 mmoles) en solution dans le THF (10 ml) est ajouté goutte à goutte, et le nouveau mélange est agité à la même température pendant 1 heure, puis abandonné à la température ambiante, pendant 15 heures. L'ensemble est versé dans l'eau, extrait au chlorure

de méthylène, et les phases organiques réunies sont séchées sur sulfate de sodium et évaporées. Le résidu huileux cristallise progressivement dans l'hexane ou le cyclohexane, et le solide obtenu est recristallisé dans le même solvant pour fournir le composé attendu, sous la forme de cristaux incolores.

Acétal (A)
Rendement : 72%
P.f. : 92°C (cyclohexane) ;
L'analyse élémentaire de l'acétal (A) ainsi obtenu donne les résultats suivants :

| | C | H | N | Cl |
|---|---|---|---|---|
| Calculé pour $C_{18}H_{20}ClNO_5$ | 59,10 | 5,51 | 3,83 | 9,69 |
| Trouvé | 58,82 | 5,47 | 4,02 | 9,67 |

Le spectre de résonance magnétique nucléaire confirme la structure attendue :
$^1H$ RMN ($CDCl_3$) δ :
1,73 (s,3H,$CH_3$),
3,72 (s,2x3H,$OCH_3$),
3,8-4,6 (m,4H,$CH_2CH_2$),
4,29 (d,1H,OH,$J_{CH-OH}$:8,2Hz),
6,66-6,84 (m,4H,CHOH + 3H-Ar)
7,57 (d, 1H,H-5 py,$J_{5-6}$ = 5Hz)
8,31 (d, 1H,H-6 py)

Acétal (B) :
Rendement : 73%
P.f. : 146°C (cyclohexane)
L'analyse élémentaire de l'acétal (B) ainsi obtenu donne les résultats suivants :

| | C | H | N | Cl |
|---|---|---|---|---|
| Calculé pour $C_{20}H_{18}ClNO_3$ | 67,51 | 5,10 | 3,94 | 9,96 |
| Trouvé | 67,71 | 5,16 | 3,96 | 9,89 |

Le spectre de résonance magnétique nucléaire confirme la structure attendue :
$^1H$ RMN ($CDCl_3$) δ :
1,77 (s,3H,$CH_3$),
3,58-3,93 (m,4H,$CH_2$,$CH_2$),
4,35 (d, 1H,OH,$J_{OH-CH}$= 11,2Hz),
6,81 (d, 1H,CH-OH),
7,41-7,50 (m,5H,5H-Ar),
7,61 (d, 1H,H-5 py,$J_{5-6}$=5Hz),
7,78-7,87 (m,2H,2H-Ar),
8,41 (d, 1H,H-6 py).

Acétal (C) :
Rendement : 61%
P.f. : 105°C (hexane)
L'analyse élémentaire de l'acétal (C) ainsi obtenu donne les résultats suivants :

| | C | H | N | S | Cl |
|---|---|---|---|---|---|
| Calculé pour $C_{14}H_{14}ClNO_3S$, 0,25$H_2O$ | 53,16 | 4,58 | 4,43 | 10,12 | 11,23 |
| Trouvé | 53,21 | 4,45 | 4,41 | 10,13 | 11,21 |

Le spectre de résonance magnétique nucléaire confirme la structure attendue :
$^1H$ RMN ($CDCl_3$) δ :
1,75 (s,3H,$CH_3$),

3,2-4,02 (m,4H,$CH_2$-$CH_2$),
4,52 (d, 1H,OH,$J_{OH-CH}$= 11,5Hz),
6,62 (dd, 1H,CHOH,$J_{CH-H-2th}$= 2Hz),
6,88-7 (m,2H,H-2th + H-4th),
7,26-7,34 (m,1H,H-5th),
7,56 (d,1H,H-5 py,$J_{5-6}$= 5 Hz),
8,37 (d, 1H,H-6 py).

c) Troisième étape :

Réduction-hydrolyse-cyclisation des acétals (A), (B) et (C) en vue de la formation des dérivés polycycliques selon l'invention, des formules respectivement :

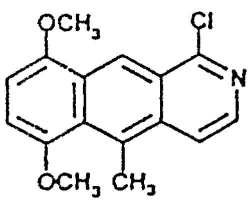

(IA)

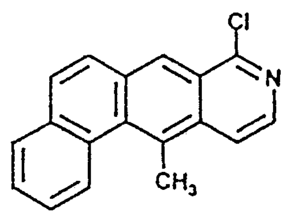

(IB)

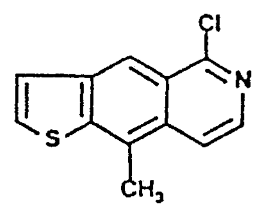

(IC)

Le composé précité (A) ou (B) ou (C) (1 mmole) est ajouté à l'acide trifluoroacétique (2 ml), et à la solution résultante agitée à une température comprise entre 10 et 25°C, le triéthylsilane (0,18 ml. 1,1 mmole) est ajouté en une seule fois. L'agitation est poursuivie pendant 18 heures à la température ambiante, et l'excès d'acide trifluoroacétique est évaporé sous pression réduite. L'acide chlorhydrique 6N (20 ml) est ajouté au résidu, et le mélange est agité à 55-60° C pendant 4 heures pour former les composés (IA) et (IB), et seulement pendant 45 minutes pour préparer la thiénoisoquinoléine (IC). Le mélange refroidi est alcalinisé par l'ammoniaque, extrait au chlorure de méthylène, et la phase organique, séchée sur sulfate de sodium, est filtrée et évaporée. Le résidu obtenu cristallise dans le minimum de méthanol (composés IA et IC) ou dans l'hexane (composé IB) et les produits obtenus sont recristallisés dans le même solvant.
(IA) : p.f. : 170° C ; rendement : 65%
(IB) : p.f. : 151° C ; rendement : 37%
(IC) : p.f. : 138° C ; rendement : 60%
Pour les composés (IB) et (IC), les points de fusion, les rf en CCM sur plaque de silice, avec plusieurs mélanges et solvants comme éluants, de même que les spectres de RMN $^1$H sont en tous points identiques à ceux des mêmes produits déjà décrits.

EXEMPLE 2 :

a) Première étape :

Préparation du formyl-3 méthoxy-5 méthyl-1 indole :

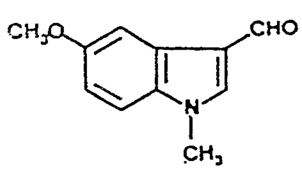

En refroidissant pour maintenir la température du milieu réactionnel au-dessous de 15° C, l'oxychlorure de phosphore (9 ml) est ajouté goutte à goutte dans 35 ml de diméthylformiate (DMF) et le méthoxy-5 méthyl-1 indole (14,1 g) en solution dans le DMF (25 ml) est ajouté en une seule fois. Le mélange hétérogène résultant est chauffé à 110° C pendant 1 minute, puis maintenu à 65° C, pendant 30 minutes, en l'agitant mécaniquement.

Après l'avoir laissé refroidir, il est décomposé par addition de 200 ml d'eau glacée, puis traité par une solution d'hydroxyde de sodium 5N (100 ml). Le précipité formé est filtré et recristallisé dans l'éthanol pour donner 14,53 g (87,7%) de paillettes incolores, p.f. = 134°C.

L'analyse élémentaire du produit obtenu donne les résultats suivants :

| | C | H | N |
|---|---|---|---|
| Calculé pour $C_{11}H_{11}NO_2$ | 69,82 | 5,86 | 7,40 |
| Trouvé | 69,84 | 7,71 | 7,4 |

Le spectre de résonance magnétique nucléaire confirme la structure attendue :
$^1$H RMN ($CDCl_3$) δ :
3,82 + 3,90 (2s,2x3H,N-$CH_3$ + $OCH_3$),
6,97 (q, 1H,H-6,$J_{6-7}$=8,3 Hz,$J_{6-4}$=2,5Hz),
7,24 (d, 1H,H-7),
7,80 (d, 1H,H-4),
9,94 (s, 1H,C<u>H</u>O).

b) Deuxième étape :

Préparation de l'acétyl-4 chloro-2 (α-méthoxy-5' méthyl-1' indolyl-3') α-hydroxy méthyl-3 pyridine éthylène glycol acétal.

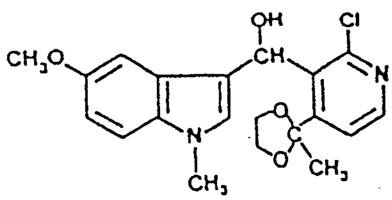

A 100 ml de THF refroidi à 0°C et maintenu sous argon sec, sont ajoutées, successivement, une solution de butyllithium 1,6N dans l'hexane (15 ml, 24 mmoles) et la diisopropylamine (3,36 ml, 24 mmoles). Le mélange est agité pendant 1 heure à 0°C, refroidi à -70°C, et l'acétyl-4 chloro-2 pyridine éthylène glycol acétal (4 g, 20 mmoles) en solution dans le THF (10 ml) est ajouté en une seule fois. Après 4 heures d'agitation à -70°C, l'aldéhyde obtenu à la première étape (3,78 g, 20 mmoles), en solution dans le THF (20 ml), est additionné progressivement, et le mélange est agité à -70°C pendant 2 heures, puis abandonné à température ambiante pendant 15 heures. Le mélange est versé dans l'eau, extrait au chlorure de méthylène, et l'évaporation du solvant fournit un résidu, qui cristallise lentement dans l'acétate d'éthyle. Le solide filtré recristallise dans le même solvant pour donner 5,04 g (65%) de microcristaux incolores, p.f. : 125°C.

L'analyse élémentaire du produit obtenu donne les résultats suivants :

| | C | H | N | Cl |
|---|---|---|---|---|
| Calculé pour $C_{20}H_{21}ClN_2O_4$ | 61,78 | 5,44 | 7,21 | 9,12 |
| Trouvé | 61,98 | 5,30 | 7,31 | 9,42 |

Le spectre de résonance magnétique nucléaire confirme la structure attendue :
$^1$H NMR ($CDCl_3$) δ :
1,73 (s,3H,$CH_3$),
3,66 (s,3H,$NCH_3$),
3,58-3,95 (m,2x2H,$CH_2CH_2$),
3,85 (s,3H,$OCH_3$),
4,24 (d,1H,O<u>H</u>,$J_{OH-CH}$: 11,6Hz),
6,39 (s,1H,H-2,Ar),
6,89 (m, 2H,C<u>H</u>-OH + H-6 Ar),
7,15-7,27 (m,2H,H-4 Ar + H-7 Ar),

7,60 (d,1H,H-5 py,$J_{5-6}$=5,3 Hz),
8,37 (d,1H,H-6 py).

c) Troisième étape :

Préparation du chloro-1 méthoxy-9 diméthyl-5,6 6H-pyrido[4,3-b]carbazole :

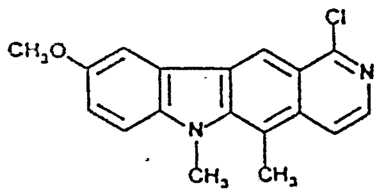

Le mélange formé par l'alcool obtenu à la deuxième étape (388 mg, 1 mmole), le triéthylsilane (0,18 ml, 1,1 mmole) et l'acide trifluoroacétique (2 ml) est agité à la température ambiante, pendant 20 heures, et évaporé à sec. Au résidu obtenu, on ajoute 5 ml d'eau et 5 ml d'acide sulfurique (d = 1,86), et le mélange est chauffé à 60°C pendant 4 heures, sous agitation. Après 15 heures à la température ambiante, on ajoute 50 ml d'eau, alcalinisé avec l'ammoniaque concentrée et on extrait au chlorure de méthylène. L'évaporation du solvant fournit un résidu qui cristallise et recristallise dans l'acétate d'éthyle, pour donner 170 mg (55%) de microcristaux jaunes, p.f. : 206°C.

Le composé ainsi obtenu est identique à celui décrit antérieurement (p.f., analyse, RMN).

$^1$H RMN ($CDCl_3$) δ =
3,07 (s,3H,$CH_3$-5),
3,99 + 4,13 (2s,2 x 3H,$OCH_3$+ $NCH_3$),
7,28-7,38 (m, 2H, H-7 + H-8),
7,72 (q,1H,H-10,$J_{10-8}$=2,5Hz,$J_{10-7}$=0,7Hz),
7,83 (q,1H,H-4,$J_{4-3}$=6,1Hz,$J_{4-11}$=1Hz),
8,21 (d,1H,H-3),
8,90 (d,1H,H-11).

EXEMPLE 3

a) Première étape :

Préparation de la formyl-3 méthyl-1 1H- pyrrolo[3,2]-pyridine

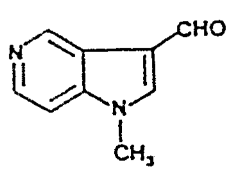

Le mélange formé par la méthyl-1 1H-pyrrolo[3,2-c]pyridine (2,64 g, 20 mmoles), l'hexaméthylène tétramine (5,6 g, 40 mmoles) et l'acide trifluoracétique (35 ml) est chauffé au reflux pendant 3 heures. Après addition d'acide chlorhydrique 3N (100 ml), le reflux est poursuivi pendant 3 heures, et le mélange est évaporé à sec. Le résidu est repris dans l'eau (75 ml), alcalinisé par une solution d'hydroxyde de sodium N, et la solution résultante, saturée par du chlorure de sodium, est extraite au chlorure de méthylène. L'évaporation du solvant laisse un solide qui recristallise dans l'acétate d'éthyle, pour fournir 2,3 g (72%) d'aiguilles incolores, p.f. = 109-110°C.

L'analyse élémentaire du produit obtenu donne les résultats suivants :

| | C | H | N |
|---|---|---|---|
| Calculé pour $C_9H_8N_2O$ | 67,50 | 5,00 | 17,50 |
| Trouvé | 67,46 | 5,08 | 17,34 |

Le spectre de résonance magnétique nucléaire confirme la structure attendue :
$^1H$ NMR ($CDCl_3$) δ :
3,83 (s,3H,$CH_3$),
7,21 (q,1H,H-7,$J_{7\text{-}6}$=5,7Hz, $J_{4\text{-}7}$=1Hz),
7,65 (s,1H,H-2),
8,42 (d,1H,H-6),
9,45 (d,1H,H-4),
9,93 (s,1H,CHO).

b) Deuxième étape :

Préparation de l'acétyl-4 chloro-2 [α-(méthyl-1′ 1H-pyrrolo[3,2-c]pyridyl-3′)α-hydroxy]méthyl-3 pyridine éthylène glycol acétal

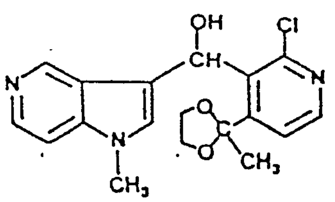

La réaction a été réalisée en appliquant le mode opératoire décrit à la deuxième étape de l'Exemple 2 avec l'aldéhyde obtenu à la première étape ci-dessus(3,2 g, 20 mmoles). Après avoir traité comme indiqué, il s'est formé 4,24 g (59%) de microcristaux incolores, p.f. = 150° C.

L'analyse élémentaire du produit obtenu donne les résultats suivants :

| | C | H | N | Cl |
|---|---|---|---|---|
| Calculé pour $C_{18}H_{18}ClN_3O_3$ | 60,09 | 5,04 | 11,68 | 9,85 |
| Trouvé | 59,92 | 5,22 | 11,62 | 9,84 |

Le spectre de résonance magnétique nucléaire confirme la structure attendue :
$^1H$ NMR ($CDCl_3$) δ :
1,74 (s,3H,$CH_3$),
3,59-3,96 (m,2x2H,$CH_2$-$CH_2$),
3,72 (s,3H,$NCH_3$),
4,35 (s large,1H,OH)
6,61 (s,1H,H-2 Ar),
6,82 (s large,1H,CH-OH),
7,20 (d,1H,H-7 Ar,$J_{6\text{-}7}$=6Hz),
7,61 (d,1H,H-5 py,$J_{5\text{-}6}$=5,5 Hz),
8,33 (d,1H,H-6 Ar),
8,38 (d,1H,H-6 py),
8,81 (s,1H-H-4 Ar).

c) Troisième étape :

Préparation de la chloro-10 diméthyl-5,6 5H-pyrido[3′,4′: 4,5] pyrrolo[2,3-g] isoquinoléine :

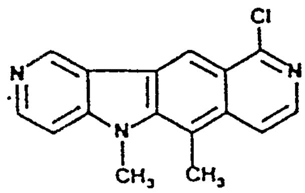

Le composé obtenu à la deuxième étape (0,720 g, 2 mmoles) est réduit, sous agitation, à la température ambiante, pendant 24 heures, dans l'acide trifluoracétique (4 ml) en présence de triéthylsilane (0,64 ml, 4 mmoles). Le résidu de l'évaporation est ensuite traité par 10 ml d'eau et 10 ml d'acide sulfurique (d = 1,86), à la température du laboratoire pendant 24 heures, sous agitation. Le mélange réactionnel est alors traité comme dans l'exemple précédent, pour fournir un solide qui recristallise dans l'acétate d'éthyle en donnant 271 mg (48%) de microcristaux jaune-pâle du composé attendu, p.f. : 256°C.

L'analyse élémentaire du produit obtenu donne les résultats suivants :

| | C | H | N | Cl |
|---|---|---|---|---|
| Calculé pour $C_{16}H_{12}ClN_3$ | 68,21 | 4,29 | 14,92 | 12,58 |
| Trouvé | 67,93 | 4,23 | 14,93 | 12,51 |

Le spectre de résonance magnétique nucléaire confirme la structure attendue :
$^1H$ RMN ($(CD_3)_2SO$) δ:
3,14 (s,3H,$CH_3$-6),
4,03 (s,3H,$NCH_3$),
7,70 (q,1H,H-4,$J_{4-3}$=5,9Hz,$J_{4-1}$=0,6Hz),
8,14 (q,1H,H-7,$J_{7-8}$=6Hz,$J_{7-11}$=0,8Hz),
8,29 (d,1H,H-8),
8,66 (d,1H,H-3),
9,14 (s élargi, 1H,H-11),
9,63 (s élargi, 1H,H-1).

EXEMPLE 4

Application du produit obtenu à l'Exemple 3 à la préparation des trois composés selon l'invention ci-après :

a) (Diéthylamino-3 propyl-1) amino-10 diméthyl-5,6 5H-pyrido [3',4':4,5] pyrrolo [2,3 g] isoquinoléine :

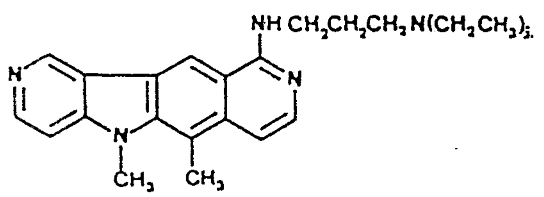

Le composé obtenu à la troisième étape de l'Exemple 3 (400 mg) est chauffé dans la diéthylamino-3 propylamine en excès (20ml), au bain d'huile, à 160°C, pendant 6 heures, et l'excès de diéthylaminopropylamine est évaporé sous pression réduite. Le résidu est repris dans l'eau, alcalinisé par une solution d'hydroxyde de sodium N et extrait au chlorure de méthylène. L'évaporation du solvant fournit une huile résiduelle qui cristallise progressivement dans le cyclohexane. Le solide obtenu recristallise dans ce solvant en donnant 476 mg (89%) de cristaux correspondant à l'hydrate du composé attendu, p.f. : 149°C.

L'analyse élémentaire du produit obtenu donne les résultats suivants :

| | C | H | N |
|---|---|---|---|
| Calculé pour $C_{23}H_{29}N_5$, $H_2O$ | 70,23 | 7,89 | 17,81 |
| Trouvé | 70,80 | 7,74 | 17,65 |

Le spectre de résonance magnétique nucléaire confirme la structure attendue :
$^1H$ RMN ($(CD_3)_2SO$): $\delta$ =
1,02 (t,2 x 3H,$(CH_2CH_3)_2$),
1,86 (m,2H,$CH_2$-β),
2,45-2,66 (m,6H,$(CH_2CH_3)_2$ + $CH_2$-γ),
2,99 (s,3H,$CH_3$-6),
3,61 (m,2H,$CH_2$-α),
4,17 (s,3H,$NCH_3$),
7,14 (d,1H,H-7,$J_{7-8}$=6,3Hz),
7,59 (s large, 1H,NH),
7,62 (q,1H,H-4,$J_{4-3}$=5,8Hz,$J_{4-1}$=0,8Hz),
8,57 (d,1H,H-3),
9,01 (s,1H,H-11),
9,29 (d,1H,H-1).

On a ensuite préparé le trimaléate acide de la (diéthylamino-3 propyl-1) amino-10 diméthyl-5,6 5H-pyrido [3',4': 4,5] pyrrolo [2,3 g] isoquinoléine (composé précédent) :

On obtient ce sel en versant une solution de la base précédente (406 mg) dans 50 ml d'acétone bouillante, dans une solution, également bouillante, de 415 mg d'acide maléique dans 50 ml du même solvant. Le mélange est maintenu à l'ébullition pendant 1 minute, refroidi, et le solide formé est essoré pour donner 736 mg (94%) de microcristaux du trimaléate acide attendu, hydraté avec 2 molécules d'eau, p.f. : 193°C.

L'analyse élémentaire de ce sel donne les résultats suivants :

| | C | H | N |
|---|---|---|---|
| Calculé pour $C_{35}H_{41}N_5O_{12}$, $2H_2O$ | 55,33 | 5,93 | 9,22 |
| Trouvé | 55,18 | 5,80 | 9,05 |

b) (Diméthylamino-3 propyl-1)-amino-10 diméthyl-5,6 5H-pyrido [3',4':4,5] pyrrolo [2,3 g] isoquinoléine :

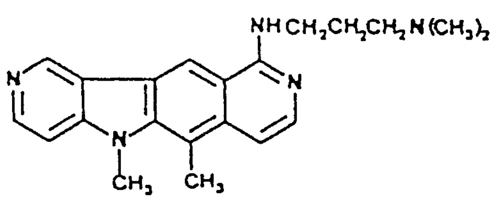

Le mélange du composé obtenu à la troisième étape de l'Exemple 3 (2 g) et de la diéthylamino-3 propylamine (100 ml) est chauffé au reflux pendant 48 heures et traité comme au paragraphe a) ci-dessus. La base cristallisée dans le cyclohexane est transformée en trimaléate acide par traitement avec un excès d'acide maléique dans l'acétone, et le sel filtré est traité par une solution d'hydroxyde de sodium en excès. Après extraction au chlorure de méthylène, la base recristallise dans le toluène, pour donner 1,14 g de cristaux. Ceux-ci sont retransformés comme déjà indiqué pour fournir 2,16 g (43,8%) de l'hydrate du trimaléate acide de la (diméthylamino-3 propyl-1)-amino-10 diméthyl-5,6 5H-pyrido [3',4':4,5] pyrrolo [2,3 g] isoquinoléine.

L'analyse élémentaire du produit obtenu donne les résultats suivants :

| | C | H | N |
|---|---|---|---|
| Calculé pour $C_{33}H_{37}N_5O_{12}$, $H_2O$ | 55,54 | 5,47 | 9,82 |
| Trouvé | 55,60 | 5,43 | 9,89 |

Le spectre de résonance magnétique nucléaire confirme la structure attendue :
$^1H$ RMN ($D_2O$) $\delta$ :
2,4 (m,2H,$CH_2$-$\beta$),
3,04 (s,2 x 3H,$N(CH_3)_2$),
3,07 (s,3H,$CH_3$-6),
3,5 (m,2H,$CH_2$-$\gamma$),
3,84 (t,2H,$CH_2$-$\alpha$),
4,3 (s,3H,$NCH_3$-5),
6,07 (s,6H,CH = CH maléate),
7,55 (d,1H,H-7,$J_{7-8}$=7,7Hz),
7,69 (d,1H,H-8),
8,05 (d,1H,H-4,$J_{4-3}$= 7Hz),
8,74 (d,1H,H-3),
9,15 (s,1H,H-11),
9,47 (s,1H,H-1).

c) (Ethylamino-3 propyl-1)-amino-10 diméthyl-5,6 5H,-pyrido[3′,4′:4,5] pyrrolo [2,3-g] isoquinoléine :

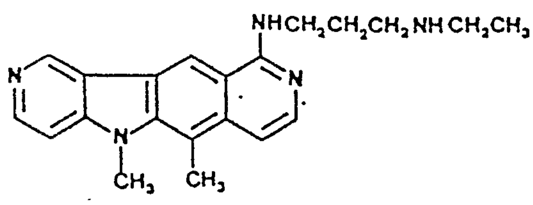

Le composé obtenu à la troisième étape de l'Exemple 3 (300 mg) est chauffé au bain d'huile, à 140°C, pendant 5 heures dans l'éthylamino-3 propylamine (15 ml), et l'excès de cette dernière est évaporé. Le résidu est repris dans l'eau, alcalinisé par une solution d'hydroxyde de sodium N et extrait au chlorure de méthylène. L'évaporation du solvant fournit une huile qui est directement traitée par un excès d'acide maléique, dans l'acétone bouillante. Il se forme ainsi, 560 mg (73,7%) de l'hydrate du trimaléate acide de l'(éthylamino-3 propyl-1)-amino-10 diméthyl-5,6 5H-pyrido[3′,4′:4,5] pyrrolo [2,3-g] isoquinoléine, p.f. : progressivement entre 165°C et 170°C.

L'analyse élémentaire de ce sel obtenu donne les résultats suivants :

| | C | H | N |
|---|---|---|---|
| Calculé pour $C_{33}H_{37}N_5O_{12}$, $H_2O$ | 55,54 | 5,47 | 9,82 |
| Trouvé | 55,37 | 5,58 | 10,17 |

Le spectre de résonance magnétique nucléaire confirme la structure attendue :
$^1H$ RMN ($D_2O$) $\delta$ :
1,35 (t,3H,$CH_2\underline{CH_3}$) ;
2,29 (m,2H,$CH_2$-$\overline{B}$) ;
2,96 (s,3H,$CH_3$-6) ;
3,24 (m,2x2H,$\underline{CH_2}CH_3$+ $CH_2$-$\gamma$) ;
3,75 (t,2H,$CH_2$-$\overline{\alpha}$) ;
4,21 (s,3H,$NCH_3$) ;
6,11 (s,6H,CH = CH-maléate) ;
7,44 (d,1H,H-7,$J_{7-8}$= 8Hz) ;

7,63 (d,1H,H-8) ;
7,90 (d,1H,H-4,$J_{4\text{-}3}$= 6,9 Hz) ;
8,65 (d,1H,H-3) ;
8,94 (s,1H,H-11) ;
9,32 (s,1H, H-1).

Les trois composés des exemples 4a), 4b) et 4c) ci-dessus, obtenus comme décrit ainsi que sous forme de sels présentent une activité antitumorale, les résultats de leur étude pharmacologique in vitro et in vivo, étant décrits dans ci-après :

1/ Essai in vitro

La méthode est celle décrite par Paoletti et col. dans ≪Chem. Biol Interaction 25, pages 45-58 (1979)≫. On ajoute des concentrations croissantes du composé à tester à une culture cellulaire de la lignée leucémie L 1210 en phase exponentielle de croissance. La culture est incubée à 37° C dans une étuve à $CO_2$ et les cellules sont comptées toutes les 24 heures. Le calcul de la $DI_{50}$, concentration du produit en micromoles par litre qui inhibe la prolifération cellulaire à 50%, est effectué après 48 heures.

La $DI_{50}$ du composé de l'exemple 4a) est de 0,017 µM ;
du composé de l'exemple 4b) est de 0,019 µM ; et
du composé de l'exemple 4c) est de 0,033 µM.

A titre de comparaison, celle du composé non substitué sur l'azote de l'indole, décrit dans la demande de brevet français n° 2 436 786, qui est étudié en clinique, est de 0,018 µM.

2/ Essai in vivo

La méthode est celle décrite par Geran et col. dans ≪Cancer Chemother, 2, pages 7-57 (1972)≫

Toutes les souris mâles ou femelles sont inoculées par voie intrapéritonéale au jour $J_0$ avec une quantité déterminée de cellules tumorales viables, variable selon la tumeur choisie pour l'essai.

Elles sont ensuite réparties en différents lots. Chaque lot correspondant aux animaux traités par le produit à tester, à une dose donnée, est composé de 10 souris. Le produit à tester est dissous dans l'eau distillée, et il est administré, pendant n jours ($J_1$-$J_n$) variables selon le protocole choisi, par voie intrapéritonéale à raison de 0,1 ml par 10 g de poids des souris traitées ; les doses du produit administré sont précisées dans les tableaux ci-dessous.

Le lot ≪contrôle négatif≫, pour lequel les animaux ne sont pas traités par un produit, comprend plus de 2 $\sqrt{N}$ animaux, N étant égal au nombre total de souris traitées par un produit.

On évalue l'activité antitumorale en considérant l'augmentation du temps de survie des animaux traités par raport à celui des animaux du lot ≪Contrôle négatif≫ selon la formule :

$$T/C = \frac{\text{jour médian de survie des animaux traités à une dose de produit}}{\text{jour médian de survie des animaux (contrôle négatif)}} \times 100$$

Un composé est considéré comme étant toxique lorsque cette valeur est égale ou inférieure à 85% ou lorsque la variation du poids des animaux, exprimée en grammes et calculée en faisant la différence entre le poids moyen du lot mesure au jour 5 et le poids moyen du même lot au jour 1 est égale ou inférieure à -4 g.

Les essais sont effectués avec des souris hybrides femelles $CD_2F_1$, inoculées par voie intrapéritonéale avec $10^6$ cellules leucémiques de la lignée P 388. Le composé à tester est administré soit une fois ($J_1$), soit pendant 5 jours ($J_1$-$J_5$) ; le produit à tester est comparé au 5-fluoro uracile (témoin positif), qui est aministré dans les mêmes conditions.

Le nombre d'animaux survivants est évalué au jour J30. Dans ces conditions, un composé est considéré comme étant actif lorsque le rapport T/C est supérieur à 128%.

Les résultats sont indiqués dans les tableaux suivants :
Tableau I pour l'administration unique à $J_1$
Tableau II pour une administration répétée.

Tableau I

| PRODUIT | DOSES mg/kg | VARIATION PONDERALE P5-P1 (g) | JOUR MEDIAN DE SURVIE | T/C % |
|---|---|---|---|---|
| Composé de l'exemple 4c) sous forme du sel obtenu | 2,5 | -1,8 | 13,8 | 118 |
| | 5 | -0,8 | 14,0 | 121 |
| | 10 | -0,2 | 14,1 | 122 |
| | 20 | -1,2 | 15,2 | 131 |
| | 40 | -2,3 | 16,0 | 138 |
| | 80 | -4,3 | 8,1 | 70 |
| Composé de l'exemple 4a) sous forme du sel obtenu | 2,5 | -VS,1¦-1,6 | 12,6 | 109 |
| | 5 | -1,0 | 13,8 | 118 |
| | 10 | -2,2 | 14,1 | 122 |
| | 20 | -1,8 | 15,4 | 133 |
| | 40 | -2,6 | 15,2 | 131 |
| | 80 | -3,5 | 17,0 | 146 |
| 5-Fluoro uracile | 200 | -2,2 | 16,0 | 138 |
| Eau distillée | | -0,9 | 11,6 | |

TABLEAU II

| PRODUIT | DOSES mg/kg | VARIATION PONDERALE P5-P1 (g) | JOUR MEDIAN DE SURVIE | T/C % |
|---|---|---|---|---|
| Composé de l'exemple 4b) sous forme du sel obtenu | 0,375 | +1,2 | 13,0 | 110 |
| | 0,75 | +0,5 | 14,8 | 125 |
| | 1,5 | +0,5 | 16,1 | 136 |
| | 3 | -0,3 | 17,0 | 144 |
| | 6 | +0,9 | 16,3 | 138 |
| | 12 | -0,7 | 17,0 | 144 |
| | 24 | -4,5 | 17,3 | 146 |
| | 48 | -4,5 | 7,2 | 61 |
| 5-Fluoro uracile | 20 | +1,0 | 20 | 169 |
| Eau distillée | | +2,6 | 11,8 | |

**Revendications**

1 - Procédé de préparation d'un composé représenté par la formule (I) ci-après :

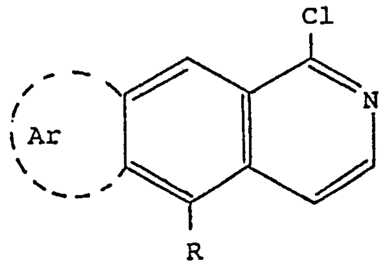

(I)

où :- R représente un groupe alkyle en $C_1$ à $C_3$ ; et
- Ar représente un groupe aromatique ou hétéroaromatique, mono- ou polycyclique, éventuellement substitué,
ainsi que les composés correspondants obtenus par substitution nucléophile du chlore du noyau pyridinique,
caractérisé par le fait qu'il comprend les étapes consistant à :
(a) effectuer une lithiation du composé de formule (II) :

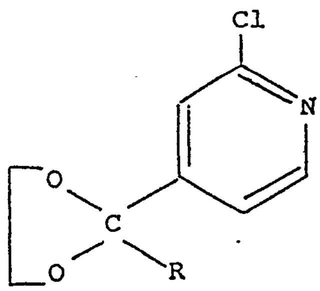

(II)

où R est tel que défini ci-dessus,
de façon à former le composé de formule (III) :

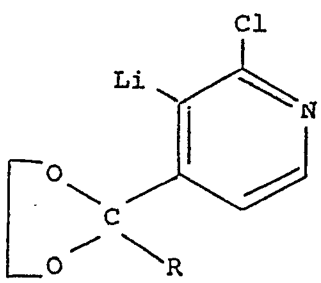

(III)

(b) faire réagir un aldéhyde de formule (IV) :
ArCHO (IV)
où Ar est tel que défini ci-dessus,
avec le composé de formule (III) précité, ce qui conduit à l'alcool secondaire de formule (V) :

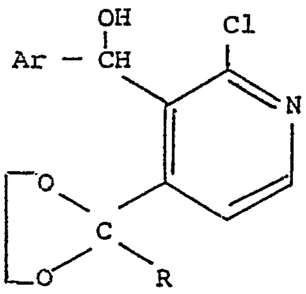

(V)

(c) on soumet l'alcool secondaire de formule (V) à une réduction de la fonction alcool secondaire en groupe méthylénique, ce qui conduit au composé de formule (VI) :

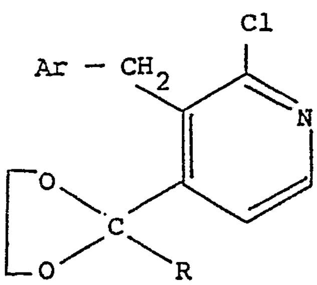

(VI) ; et

et
(d) on soumet le composé de formule (VI) à une réaction d'hydrolyse (de la fonction dioxolanne)-cyclisation-déshydratation, ce qui conduit au composé de formule (I) ;
et l'on peut faire suivre par une réaction de substitution nucléophile du chlore.

2 - Procédé selon la revendication 1, caractérisé par le fait que l'on conduit l'étape (a) de lithiation du composé de formule (II) par action du diisopropylamidure de lithium dans le tétrahydrofuranne anhydre.

3 - Procédé selon l'une des revendications 1 et 2, caractérisé par le fait que l'on conduit l'étape (b) de condensation du composé organolithien de formule (III) avec l'aldéhyde de formule (IV), par addition de ce dernier à une solution, notamment dans le tétrahydrofuranne anhydre, dudit composé organolithien de formule (III).

4 - Procédé selon l'une des revendications 1 à 3, caractérisé par le fait que l'on conduit la réaction de réduction de l'étape (c) avec utilisation du triéthylsilane en solution dans l'acide trifluoracétique.

5 - Procédé selon l'une des revendications 1 à 4, caractérisé par le fait que l'on conduit la triple réaction de l'étape (d) en milieu aqueux par action d'un acide fort, à une température comprise entre 40°C et 70°C.

6 - Procédé selon l'une des revendications 1 à 5, caractérisé par le fait que l'on n'isole pas le composé de formule (VI).

7 - Procédé selon l'une des revendications 1 à 6, caractérisé par le fait que l'on prépare des composés de formule (I) dans laquelle Ar représente :

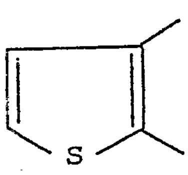 ; 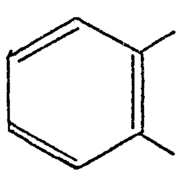 ; 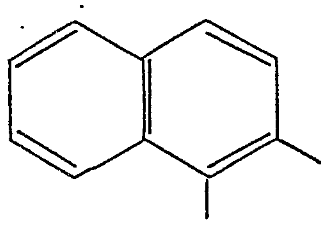 ; 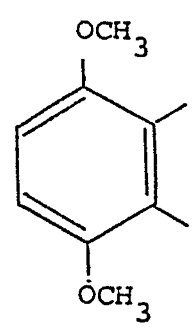

8 - Procédé selon l'une des revendications 1 à 6, caractérisé par le fait que l'on prépare des composés de formule (I')

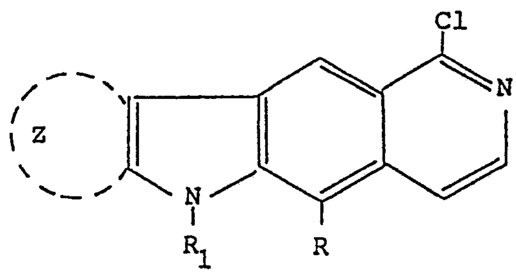

(I')

où :

- R représente un groupe alkyle en $C_1$ à $C_3$ ;
- $R_1$ représente un groupe alkyle en $C_1$-$C_3$ ou un groupement protecteur éliminable ; et
- Z représente un groupe aromatique ou hétéroaromatique, substitué ou non substitué, tel que :

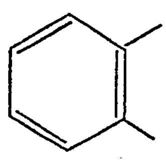 , 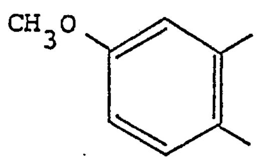 , 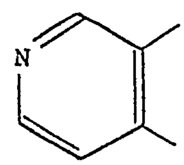 ,

auquel cas, on fait réagir, comme composé de formule (IV), l'aldéhyde de formule (IVa) N-protégé :

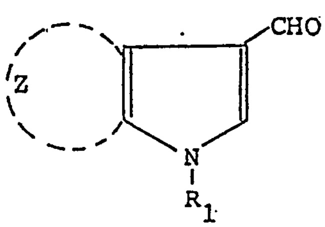

(IVa)

où $R_1$ est tel que défini ci-dessus.

9 - Composés de formule (I) tels que définis à la revendication 1, dans laquelle Ar représente :

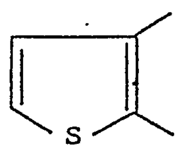 , 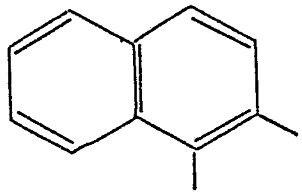 , 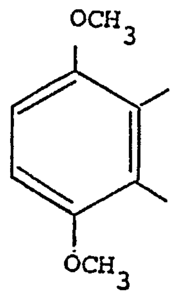

et notamment 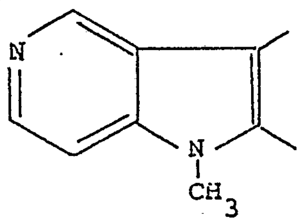 .

10 - Composés selon la revendication 9, caractérisés par le fait qu'ils sont représentés par la formule :

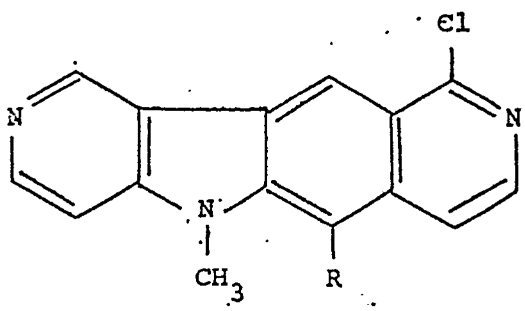

où R est tel que défini à la revendication 1, et les composés résultant de la substitution nucléophile du chlore.

11 - Composés selon la revendication 11, caractérisés par le fait qu'ils sont représentés par la formule :

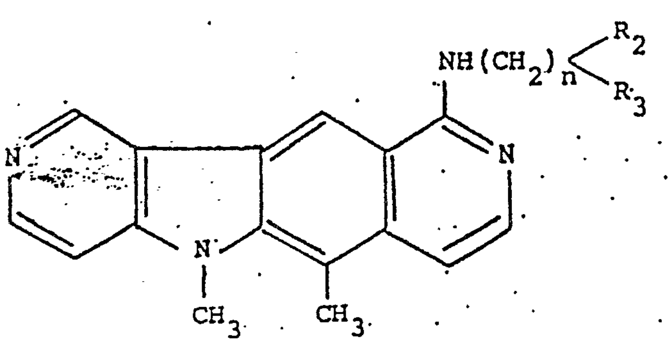

où :
- n = 2 à 4 ; et
- $R_2$ et $R_3$ représentent chacun indépendamment l'un de l'autre, H ou alkyle en $C_1$-$C_3$.

12 - Composition pharmaceutique, caractérisée par le fait qu'elle comprend, dans un support pharmaceutiquement acceptable, au moins un composé tel que défini à la revendication 11.

13 - Composition selon la revendication 12, caractérisée par le fait qu'elle est destinée au traitement des cancers.

14 - Composition selon l'une des revendications 12 et 13, caractérisée par le fait qu'elle se présente sous une forme administrable par la voie intraveineuse ou la voie intrapéritonéale.

Office européen des brevets

# RAPPORT DE RECHERCHE EUROPEENNE

Numero de la demande

EP 88 40 2852

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | EP-A-0 010 029 (ANVAR)<br>* Page 1, lignes 8-12; revendications *<br>--- | 9-14 | C 07 D 221/08<br>C 07 D 471/04<br>C 07 D 471/14<br>C 07 D 495/04<br>A 61 K 31/47<br>A 61 K 31/475<br>C 07 D 221/18 |
| D,A | FR-A-2 422 662 (ANVAR)<br>* Pages 1,20,24,25 *<br>----- | 9-14 | |
| | | | **DOMAINES TECHNIQUES RECHERCHES (Int. Cl.4)** |
| | | | C 07 D 221/00<br>C 07 D 471/00<br>C 07 D 495/00<br>C 07 D 405/00 |

Le présent rapport a été établi pour toutes les revendications

| Lieu de la recherche | Date d'achèvement de la recherche | Examinateur |
|---|---|---|
| LA HAYE | 15-12-1988 | FRANCOIS J.C.L. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)